(19) Europäisches Patentamt
European Patent Office
Office européen des brevets

(11) EP 1 448 502 B1

(12) # EUROPÄISCHE PATENTSCHRIFT

(45) Veröffentlichungstag und Bekanntmachung des Hinweises auf die Patenterteilung:
**14.10.2015 Patentblatt 2015/42**

(21) Anmeldenummer: **01274735.8**

(22) Anmeldetag: **15.11.2001**

(51) Int Cl.:
***C07C 29/80*** *(2006.01)* ***C07C 29/88*** *(2006.01)*
***C07C 31/22*** *(2006.01)*

(86) Internationale Anmeldenummer:
**PCT/EP2001/013205**

(87) Internationale Veröffentlichungsnummer:
**WO 2003/043963 (30.05.2003 Gazette 2003/22)**

(54) **VERFAHREN ZUM ENFERNEN VON FORMALDEHYDHALTIGEN ACETALEN AUS MEHRWERTIGEN ALKOHOLEN DURCH TEMPERN**

METHOD FOR REMOVING ACETALS CONTAINING FORMALDEHYDE FROM POLYVALENT ALCOHOLS BY MEANS OF TEMPERING

PROCEDE PERMETTANT DE SUPPRIMER LES ACETALS CONTENANT DE L'ALDEHYDE FORMIQUE D'ALCOOLS POLYVALENTS PAR RECUISSON

(84) Benannte Vertragsstaaten:
**AT BE CH CY DE DK ES FI FR GB GR IE IT LI LU MC NL PT SE TR**

(43) Veröffentlichungstag der Anmeldung:
**25.08.2004 Patentblatt 2004/35**

(73) Patentinhaber: **BASF SE**
**67056 Ludwigshafen (DE)**

(72) Erfinder:
- **WARTINI, Alexander**
  **69120 Heidelberg (DE)**
- **SIRCH, Tilman**
  **67105 Schifferstadt (DE)**
- **DERNBACH, Matthias**
  **69221 Dossenheim (DE)**
- **MELDER, Johann-Peter**
  **67459 Böhl-Iggelheim (DE)**

(56) Entgegenhaltungen:
**EP-A- 1 090 899** **WO-A-98/28253**
**WO-A1-01/47847** **US-A- 4 401 834**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 448 502 B1

## Beschreibung

Die vorliegende Erfindung bezieht sich auf ein Verfahren, bei dem formaldehydhaltige Acetale in mehrwertigen Alkoholen, die durch Kondensation von Formaldehyd mit höheren Aldehyden dargestellt wurden, durch Erhitzen auf eine jeweils geeignete Temperatur (sogenanntes Tempern), zersetzt werden und auf diese Weise mehrwertige Alkohole mit geringen Acetalgehalten hergestellt werden.

Mehrwertige Alkohole werden in großem Maßstab durch Kondensation von Formaldehyd mit höheren, CH-aciden Aldehyden oder mit Wasser und Acrolein bzw. 2-Alkylacroleinen erhalten. Dabei unterscheidet man bei dieser Reaktion zwischen zwei prinzipiellen Durchführungsvarianten.

Zum einen ist dies das sogenannte Cannizzaro-Verfahren, das wiederum unterteilt wird in das anorganische und das organische Cannizzaro-Verfahren. Bei der anorganischen Variante setzt man einen Überschuß an Formaldehyd mit dem entsprechenden Alkanal in Gegenwart von stöchiometrischen Mengen einer anorganischen Base wie NaOH oder $Ca(OH)_2$ um. Das in der ersten Stufe gebildete Dimethylolbutanal reagiert in der zweiten Stufe mit dem überschüssigen Formaldehyd in einer Disproportionierungsreaktion zu Trimethylolpropan und dem Formiat der entsprechenden Base, also etwa zu Natrium- oder Kalziumformiat. Der Anfall dieser Salze stellte einen Nachteil dar, da sie schwierig vom Reaktionsprodukt abzutrennen sind, und außerdem ein Äquivalent Formaldehyd verloren geht.

Bei dem organischen Cannizzaro-Verfahren wird anstelle einer anorganischen Base ein tertiäres Alkylamin eingesetzt. Damit lassen sich höhere Ausbeuten erzielen als mit einer anorganischen Base. Es fällt als unerwünschtes Nebenprodukt Trialkylammoniumformiat an. Somit geht auch hier ein Äquivalent des Formaldehyds verloren.

Die Nachteile des Cannizzaro-Verfahrens werden bei dem sogenannten Hydrierverfahren vermieden. Dabei bringt man Formaldehyd mit dem entsprechenden Aldehyd in Gegenwart von katalytischen Mengen eines Amins zur Reaktion. Damit wird erreicht, daß die Reaktion auf der Stufe des alkylolierten Aldehyds anhält. Nach Abtrennung des Formaldehyds wird das Reaktionsgemisch, das neben dem erwähnten alkylolierten Aldehyd noch geringe Mengen des entsprechenden mehrwertigen Alkohols und von Acetalen der gebildeten Alkohole enthält, einer Hydrierung unterworfen, bei der der gewünschte mehrwertige Alkohol erhalten wird.

Ein besonders effektives Verfahren zur Herstellung von durch Kondensation von Aldehyden mit Formaldehyd erhältlichen Alkoholen wird dabei in der WO 98/28253 beschrieben. Hohe Ausbeuten, verbunden mit den Anfallen geringer Mengen an Koppelprodukten, werden mit diesem Verfahren ermöglicht. Es wird dabei so verfahren, daß der höhere Aldehyd mit der 2- bis 8-fachen Menge Formaldehyd in Gegenwart eines tertiären Amins umgesetzt wird, und man das so erhaltene Reaktionsgemisch in zwei Lösungen auftrennt, wobei eine Lösung das erwähnte vollständig methylolierte Alkanal und die andere Lösung nicht umgesetztes Ausgangsprodukt aufweist. Diese letzte Lösung wird in die Reaktion zurückgeführt. Die Auftrennung erfolgt durch Destillation oder einfaches Abtrennen der wässrigen von der organischen Phase. Die das Produkt enthaltende Lösung wird einer katalytischen und/oder thermischen Behandlung unterworfen, um nicht-vollständig alkylolierte Alkanale in die gewünschten vollständig methylolierten Verbindungen zu überführen. Hierbei entstandenes Nebenprodukt wird durch Destillation abgetrennt, und der so erhaltene Sumpf wird der katalytischen Hydrierung, die zu den mehrwertigen Alkoholen führt, unterworfen.

Beispiele für wichtige, mit den beschriebenen Verfahren hergestellte Alkohole sind Neopentylglycol, Pentaerythrit, Trimethylolethan, Trimethylolbutan und insbesondere Trimethylolpropan (TMP).

Sowohl nach dem Cannizzaro- als auch nach dem Hydrierverfahren dargestellte Alkohole müssen destillativ von Komponenten befreit werden, die leichter flüchtig sind (sogenannte Leichtsieder) bzw. schwerer flüchtig sind als dieser (sogenannte Hochsieder) als auch von im Bereich des Alkohols siedenden Komponenten (sogenannte Mittelsieder). Leichtsieder sind dabei insbesondere Wasser, Methanol und bei Verwendung eines Amins als Katalysator das freie Amin.

Bei den Hochsiedern und Mittelsiedern handelt es sich oft um Verbindungen, die Derivate des hergestellten mehrwertigen Alkohols sind und aus diesem durch Reaktion mit beispielsweise Formaldehyd, Methanol oder auch einem weiteren Molekül des hergestellten Alkohols entstanden sind.

Für die Anwendung des mehrwertigen Alkohols ist insbesondere ein niedriger Gehalt des Alkohols an formaldehydhaltigen Acetalen von Bedeutung.

Unter formaldehydhaltigen Acetalen werden dabei alle Verbindungen verstanden, die sich von Formaldehyd ableiten und das Strukturelement

$$-O-CH_2-O- \qquad (I)$$

aufweisen und auch als Formale bezeichnet werden können.

Bei der Herstellung mehrwertiger Alkohole treten formaldehydhaltige Acetale der allgemeinen Formeln (IIa) oder (IIb)

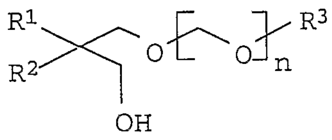
(IIa)

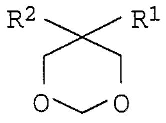
(IIb)

auf, in denen

$R^1$, $R^2$ unabhängig voneinander Wasserstoff, $C_1$- bis $C_{10}$-Alkyl, $C_1$-bis $C_{10}$-Hydroxyalkyl, Carboxyl oder $C_1$- bis $C_4$-Alkoxycarbonyl, bevorzugt $C_1$- bis $C_{10}$-Alkyl und $C_1$- bis $C_{10}$-Hydroxyalkly,

$R^3$ Wasserstoff, $C_1$- bis $C_{10}$-Alkyl, bevorzugt $C_1$- bis $C_8$-, besonders bevorzugt $C_1$- bis $C_5$-Alkyl, oder $C_1$- bis $C_{10}$-Hydroxyalkyl, bevorzugt $C_1$- bis $C_8$-, besonders bevorzugt $C_1$- bis $C_5$-Alkyl, und

n eine ganze Zahl von 1 bis 4, bevorzugt von 1 bis 3 und besonders bevorzugt 1 bis 2,

bedeuten und die Alkylreste jeweils verzweigt oder unverzweigt sein können.

Beispiele für $R^1$ und $R^2$ sind Wasserstoff, Methyl, Ethyl, iso-Propyl, n-Propyl, n-Butyl, iso-Butyl, sek-Butyl, tert-Butyl, n-Pentyl, n-Hexyl, n-Heptyl, n-Octyl, n-Decyl, Hydroxymethyl, Carboxyl, Methoxycarbonyl, Ethoxycarbonyl oder n-Butoxycarbonyl, bevorzugt Wasserstoff, Hydroxymethyl, Methyl und Ethyl, besonders bevorzugt Hydroxymethyl, Methyl und Ethyl.

Beispiele für $R^3$ sind Wasserstoff, Methyl, Ethyl, n-Propyl, n-Butyl, 2-Methylpropyl, 2-Methylbutyl, 2-Ethyl-3-hydroxypropyl, 2-Methyl-3-hydroxypropyl, 2,2-Bis(hydroxymethyl)butyl, 2,2-Bis(hydroxymethyl)propyl, 2,2-Dimethyl-3-hydroxypropyl, 3-Hydroxypropyl, 3-Hydroxy-2-(hydroxymethyl)propyl oder 3-Hydroxy-2,2-bis(hydroxymethyl)propyl.

Typische formaldehydhaltige Acetale sind beispielsweise für den Fall der Synthese des dreiwertigen Alkohols Trimethylolpropan (TMP) aus Formaldehyd und n-Butyraldehyd in Gegenwart katalytischer Mengen an Trialkylamin die nachfolgend genannten TMP-Formaldehyd-Methanol-Acetale (IIIa) und (IIIb), welche im Rohprodukt des Hydrierverfahren zu 0,05 bis 10 Gew.-% enthalten sein können,

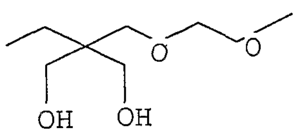
(IIIa)

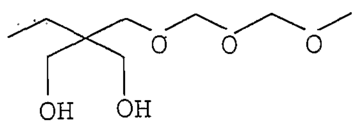
(IIIb)

aber auch das lineare bis-TMP-Formal $[C_2H_5C(CH_2OH)_2CH_2O]CH_2$ (IV) und das cyclische TMP-Formal

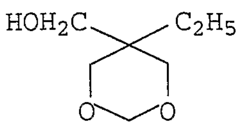
(V).

Es ist offensichtlich, dass die Bildung dieser Einheiten des mehrwertigen Alkohols, insbesondere TMP-Einheiten, enthaltenden Acetale unerwünscht ist, da sie die Ausbeute an gewünschtem Produkt deutlich senken und zudem die Anwendungseigenschaften des Produktalkohols negativ beeinflussen. Um diese Nachteile zu vermeiden, ist es wünschenswert, die formaldehydhaltigen Acetale zu entfernen. Dabei werden in der Literatur verschiedene Verfahren offenbart, um dieses zu erreichen.

In US 6 096 905 ist ein Verfahren offenbart, bei dem eine das lineare bis-TMP-Formal oder das lineare bis-Trimethylolethanformal enthaltende Zusammensetzung mit einem stark sauren Katalysator bei 30 bis 300°C 1/2 bis 8 Stunden lang behandelt wird.

GB-A 1 290 036 beschreibt ein Verfahren, bei dem eine nach dem anorganischen Cannizzaro-Verfahren erhaltene TMP-Rohlösung, mit einem Kationentauscher behandelt wird.

Nachteilig an beiden aus dem Stand der Technik bekannten Verfahren ist es, dass das saure Medium zu Nebenreaktionen führen kann, welche Eigenschaften des gewünschten mehrwertigen Alkohols, wie die Farbzahl nachteilig beeinflussen können.

Die der vorliegenden Erfindung zugrundeliegende Aufgabe ist daher, die Bereitstellung eines Verfahrens, das es erlaubt, mehrwertige Alkohole, insbesondere TMP, mit geringem Gehalt an formaldehydhaltigen Acetalen zu erhalten. Das neue Verfahren soll auch die Aufarbeitung von Rohlösungen, der mehrwertigen Alkohole, die einen Produktgehalt von 60 bis 95 Gew.-% und einen Wassergehalt kleiner 5 Gew.-% aufweisen, ermöglichen.

Die Aufgabe wird gelöst durch ein Verfahren zum Entfernen von formaldehydhaltigen Acetalen aus mehrwertigen Alkoholen. Das Verfahren ist dadurch gekennzeichnet, dass der mehrwertige Alkohol nach seiner Herstellung durch Destillation von Leichtsiedern befreit wird, anschließend der Temperung unterworfen und anschließend erneut durch Destillation gereinigt wird.

Erfindungsgemäß wurde erkannt, dass im Fall des Trimethylolpropans aus den TMP-Formaldehyd-Methanol-Acetalen der allgemeinen Formeln IIa und IIb, die zu den Mittelsiedern gezählt werden, durch den erfindungsgemäßen Temperschritt neben TMP das cyclische TMP-Formal, das schwersiedende lineare bis-TMP-Formal und Methanol gebildet werden. Durch den Temperschritt wird so ein Gemisch mit einem leichtsiedenden Anteil (cyclisches TMP-Formal, Methanol) und Schwersiedern gebildet, das sich destillativ erheblich leichter vom Produktalkohol abtrennen lässt.

Es wurde weiterhin gefunden, dass es vorteilhaft ist, Rohlösungen der mehrwertigen Alkohole mit Produktgehalten von 60 bis 95 Gew.-%, bevorzugt 75 bis 85 Gew.-% Produktgehalt, und Wassergehalten kleiner 5 Gew.-% nach dem erfindungsgemäßen Verfahren zu behandeln. Die vorstehend genannten Rohlösungen sind dadurch erhältlich, dass nach der Synthese des mehrwertigen Alkohols lediglich die Leichtsieder wie Wasser, Methanol und das als Katalysator verwendete freie Amin abgetrennt werden.

Durch die frühzeitige Entfernung der formaldehydhaltigen Acetale wird die weitere Aufarbeitung wesentlich erleichtert. Mehrwertige Alkohole mit Gehalten an gebundenem Formaldehyd (FA-Zahl) von kleiner als 1000 Gew.-ppm, bevorzugt kleiner 500 Gew.-ppm, werden zugänglich. Es ist natürlich auch möglich, bereits destillierten mehrwertigen Alkohol mit Produktgehalten > 95 Gew.-% einzusetzen. Wenn TMP in den erfindungsgemäßen Temperschritt eingesetzt wird, ist auch der Einsatz von TMP-Lösungen mit einem Gehalt von > 98 % möglich. Durch den Einsatz bereits destillierten mehrwertigen Alkohols sind besonders niedrige Farbzahlen erreichbar.

Das erfindungsgemäße Verfahren kann dabei zur Entfernung von formaldehydhaltigen Acetalen aus mehrwertigen Alkoholen, insbesondere TMP, jeglicher Herkunft verwendet werden. Es können Chargen eingesetzt werden, die dem organischen oder dem anorganischen Cannizarro-Verfahren entstammen. Die besten Ergebnisse wurden erhalten, wenn in dem der Acetalzersetzung dienenden erfindungsgemäßen Temperschritt Alkohole eingesetzt wurden, die aus dem Hydrierverfahren stammen. Es ist dabei in jedem Fall wichtig, daß der Alkohol vorher von Leichtsiedern befreit wurde und eine Reinheit aufweist, die in dem oben genannten Bereich liegt.

Der erfindungsgemäße Temperschritt ist insbesondere auf alle mehrwertigen Alkohole anwendbar, die durch Kondensation von Formaldehyd mit höheren Aldehyden unter Zugabe katalytischer Mengen Trialkylamin und nachfolgender Hydrierung hergestellt werden können. Geeignete höhere Aldehyde sind praktisch alle Alkanale mit einem aciden Wasserstoffatom in $\alpha$-Stellung zur Carbonylgruppe. Es können aliphatische Aldehyde mit 2 bis 24 C-Atomen als Ausgangsmaterialien verwendet werden, die geradkettig oder verzweigt sein oder auch alicyclische Gruppen enthalten können. Ebenso sind araliphatische Aldehyde als Ausgangsstoffe geeignet, vorausgesetzt daß sie eine Methylengruppen in $\alpha$-Stellung zur Carbonylgruppe enthalten. Im allgemeinen werden Aralkylaldehyde mit 8 bis 24 C-Atomen, vorzugsweise mit 8 bis 12 C-Atomen als Ausgangsmaterialien verwendet, beispielsweise Phenylacetaldehyd. Bevorzugt werden aliphatische Aldehyde mit 2 bis 12 C-Atomen, beispielsweise 3-Ethyl-, 3-n-Propyl-, 3-Isopropyl-, 3-n-Butyl-, 3-Isobutyl-, 3-sek.-Butyl-, 3-tert.-Butyl-butanal sowie entsprechende n-pentanale, -n-hexanale,-n-heptanale; 4-Ethyl-, 4-n-Propyl-, 4-Isopropyl-, 4-n-Butyl-, 4-Isobutyl-, 4-sek.-Butyl-, 4-tert.-Butyl-pentanale, -n-hexanale, -n-heptanale; 5-Ethyl-, 5-n-Propyl-, 5-Isopropyl-, 5-n-Butyl-, 5-Isobutyl-, 5-sek.-Butyl-, 5-tert.-Butyl-n-hexanale, -n-heptanale; 3-Methyl-hexanal, 3-Methyl-heptanal; 4-Methyl-pentanal, 4-Methyl-heptanal, 5-Methyl-hexanal, 5-Methylheptanal; 3,3,5-Trimethyl-n-pentyl-, 3,3-Diethylpentyl-, 4,4-Diethylpentyl-, 3,3-Dimethyl-n-butyl-, 3,3-Dimethyl-n-pentyl-, 5,5-Dimethylheptyl-, 3,3-Dimethylheptyl-, 3,3,4-Trimethylpentyl, 3,4-Dimethylheptyl-, 3,5-Dimethylheptyl-, 4,4-Dimethylheptyl-, 3,3-Diethylhexyl-, 4,4-Dimethylhexyl-, 4,5-Dimethylhexyl-, 3,4-Dimethylhexyl-, 3,5-Dimethylhexyl-, 3,3-Dimethylhexyl-, 3,4-Diethylhexyl-, 3-Methyl-4-ethylpentyl, 3-Methyl-4-ethylhexyl-, 3,3,4-Trimethylpentyl-, 3,4,4-Trimethylpentyl-, 3,3,4-Trimethylhexyl-, 3,4,4-Trimethylhexyl-, 3,3,4,4,-Tetramethylpentylaldehyd; insbesondere $C_2$ bis $C_{12}$-n-Alkanale.

Besonders bevorzugte mehrwertige Alkohole im Rahmen der vorliegenden Erfindung sind Trimethylolethan, Trimethylolpropan, Trimethylolbutan, Dimethylolpropan, 1,1-, 1,2-, 1,3- und 1,4-Cyclohexandimethanol, 2-Ethyl-1,3-propandiol, 2-Methyl-1,3-propandiol, Glycerin, Neopentylglykol, Pentaerythrit und Dipentaerythrit. Der meistbevorzugte Alkohol ist Trimethylolpropan.

Sollen mit dem erfindungsgemäßen Verfahren formaldehydhaltige Acetale aus Rohlösungen mehrwertiger Alkohole, insbesondere TMP mit Produktgehalten von 60 bis 95 Gew.-% entfernt werden, wird das nach dem Hydrierverfahren erhaltene Rohprodukt (der Hydrieraustrag) vor dem Temperschritt einer Entwässerung unterzogen, bei der Wasser und andere Leichtsieder wie Methanol und Trialkylamin oder Trialkylammoniumformiat durch Destillation abgetrennt werden.

Sollen mit dem erfindungsgemäßen Verfahren formaldehydhaltiges Acetal aus nach dem Hydrierverfahren hergestelltem TMP hoher Reinheit (Produktgehalt > 98 Gew.-%) entfernt werden, kann dieses hochreine TMP nach dem in der deutschen Anmeldung mit dem Titel "Verfahren zur Reinigung von durch Hydrierung hergestelltem Trimethylolpropan durch kontinuierliche Destillation" (Anmelderin: BASF AG, Aktenzeichen 199 63 435.1) beschriebenen Verfahren hergestellt werden. Dabei wird das nach Hydrierung erhaltene Rohprodukt zunächst einer Entwässerung unterzogen, bei der Wasser und andere Leichtsieder wie Methanol, Trialkylamin oder Trialkylammoniumformiat durch Destillation abgetrennt werden. Diese Destillation kann dabei bei Drücken < 400 mbar, vorzugsweise 20 bis 200 mbar, Sumpftemperaturen < 200°C und kurzen Verweilzeiten so durchgeführt werden, daß das gebildete Trialkylammoniumformiat mit TMP nur in geringem Maß zu TMP-Formiaten und Trialkylamin abreagiert. Es ist auch möglich, die Destillation bei Drücken von > 200 mbar, vorzugsweise > 400 mbar, Sumpftemperaturen von > 140°C und langen Verweilzeiten so durchzuführen, daß zumindest der überwiegende Teil des TMP mit Trialkylammoniumformiat zu TMP-Formiaten und Trialkylamin reagiert.

Anschließend werden im nächsten Schritt die Hochsieder abgetrennt. Dies geschieht dadurch, daß vom Sumpf bei 210 bis 250°C diejenigen Komponenten durch Destillation abgetrennt werden, die bei diesen Temperaturen flüchtig sind. Die Hochsieder verbleiben somit im Sumpf. Die erhaltene, leichtsiedende TMP-reiche Fraktion wird anschließend destillativ aufgearbeitet (erste Reindestillation), wobei unerwünschte Leichtsieder abgetrennt werden. Das erhaltene Reinprodukt kann einer zweiten Reindestillation unterworfen werden, um ein besonders sauberes Produkt zu erhalten.

Es kann TMP verwendet werden, das der ersten Reindestillation entstammt, aber auch solches, das der zweiten Reindestillation entnommen wurde.

Der Inhalt der erwähnten deutschen Anmeldung ist ein wichtiger und integraler Teil der vorliegenden Erfindung und durch Referenz in die vorliegende Anmeldung eingeschlossen.

Durch den erfindungsgemäßen Temperschritt, der generell von einer Destillation gefolgt wird, lassen sich mehrwertige Alkohole mit niedrigen Gehalten an formaldehydhaltigen Acetalen herstellen. Insbesondere läßt sich TMP mit gebundenen Formaldehydgehalten kleiner 1000 Gew.-ppm und Farbzahlen von bis zu ≤ 30 APHA erhalten.

TMP, das entsprechend der deutschen Anmeldung mit dem Aktenzeichen 199 63 435.1 aufgearbeitet und der ersten Reindestillation unterzogen wurde, weist generell Farbzahlen von 40 bis 150 APHA auf. Diese wurden durch das Tempern gemäß der vorliegenden Erfindung auf Farbzahlen < 20 APHA verbessert.

Damit bei dem erfindungsgemäßen Temperschritt die gewünschten Gehalte an formaldehydhaltigen Acetalen erzielt werden, müssen bestimmte Reaktionsbedingungen eingehalten werden, die in Abhängigkeit von etwa der Art des eingesetzten mehrwertigen Alkohols, den Reinheiten der eingesetzten Produkte, den verwendeten Apparaturen und eventuell weiteren vorhandenen Zusatzstoffen variieren können. Diese Reaktionsbedingungen sind dem Fachmann durch Versuche zugänglich. Generell wird der erfindungsgemäße Temperschritt bei Temperaturen von 100 bis 300°C, vorzugsweise 160 bis 240°C, Verweilzeiten von 5 min bis 24 h, vorzugsweise 15 min bis 4 h und Drücken von 100 mbar bis 200 bar vorzugsweise 1 bis 10 bar durchgeführt. Wenn der zu reinigende mehrwertige Alkohol TMP ist, wird der erfindungsgemäße Temperschritt bei Temperaturen von 100 bis 300°C, vorzugsweise 160 bis 240°C, Verweilzeiten von 5 min bis 24 h, vorzugsweise 15 min bis 4 h, und den vorstehend erwähnten Drücken durchgeführt.

Die üblichen, dem Fachmann bekannten Apparaturen können zur Durchführung des erfindungsgemäßen Temperschritts verwendet werden, wobei dieser kontinuierlich oder diskontinuierlich durchgeführt werden kann. Bei der diskontinuierlichen Durchführung (Batch-Fahrweise) wird das erfindungsgemäße Verfahren vorzugsweise in einem Rührbehälter durchgeführt, bei der kontinuierlichen Fahrweise in einem Rohrreaktor, wobei hier auf eine Sumpf- oder Rieselfahrweise zurückgegriffen werden kann.

Die meistbevorzugte Ausführungsform des erfindungsgemäßen Temperns ist das kontinuierliche Durchführen in einem Rohrreaktor in Sumpffahrweise.

Bei allen diesen Durchführungsvarianten kann der Reaktionsbehälter mit den üblichen, einem Fachmann bekannten Schütt-Füllkörpern, beispielsweise Raschig- oder Pall-Ringen oder Packungen, wie beispielsweise Blechpackungen, versehen sein, um eine bessere Durchmischung der Komponenten zu erreichen. Auch können Träger und/oder Katalysatoren in den üblichen Konfektionierungen, beispielsweise Strängen oder Tabletten, vorhanden sein, um die bei dem erfindungsgemäßen Temperschritt ablaufenden Reaktionen zu beschleunigen. Beispiele für geeignete Träger/Katalysatoren umfassen $TiO_2$, $Al_2O_3$, $SiO_2$, geträgerte $H_3PO_4$ und Zeolithe.

Durch den erfindungsgemäßen Temperschritt werden die mittelsiedenden Acetale IIa und IIb in leicht und schwersiedende Komponenten überführt.

Der Produktalkohol läßt sich von den entstehenden leichtsiedenden und hochsiedenden schwerflüchtigen Komponenten durch Destillation leicht abtrennen. Der erfindungsgemäße Temperschritt wird daher generell von einer Destillation gefolgt werden. Da die in dem erfindungsgemäßen Temperschritt aus den Acetalkomponenten Ia und Ib entstehenden schwerflüchtigen Verbindungen sich im allgemeinen hinsichtlich ihres Siedeverhaltens deutlich von den Produktalkoholen unterscheiden, können diese durch einfache, nur eine geringe Trennwirkung aufweisende destillative Maßnahmen bzw. Methoden abgetrennt werden. Oftmals genügen dabei Trenneinheiten mit nur einer Destillationsstufe, beispielsweise Fallfilmverdampfer oder Dünnfilmverdampfer. Gegebenenfalls, insbesondere wenn die Destillation auch

der weiteren Reinigung des Produktalkohols dient, werden aufwendigere Trennverfahren bzw. Trennapparaturen zum Einsatz kommen, generell Kolonnen mit mehreren Trennstufen, beispielsweise Füllkörperkolonnen, Glockenbodenkolonnen oder Pakkungskolonnen.

Bei der Destillation werden die üblichen, einem Fachmann bekannten Bedingungen hinsichtlich Druck und Temperatur eingehalten werden, wobei diese selbstverständlich auch von dem eingesetzten Produktalkohol abhängig sind.

Auch dabei werden natürlich entsprechend den Anforderungen Apparaturen mit geringer oder hoher Trennleistung eingesetzt werden.

Wie bereits erwähnt, eignet sich bei TMP als Produktalkohol für den Einsatz in dem Verfahren nach der vorliegenden Erfindung ein TMP, das entsprechend dem in der deutschen Patentanmeldung mit dem Aktenzeichen 199 63 435.1 offenbarten Verfahren gereinigt wurde. Dabei kann ein TMP verwendet werden, das der ersten Reindestillation oder auch der zweiten Reindestillation entstammt. Kommt dieses Verfahren zur Anwendung, dann ist es nach einer Variante der Erfindung besonders bevorzugt, wenn der Temperschritt nach der ersten Reindestillation durchgeführt wird, in den üblichen Apparaturen. Daran schießt sich die zweite Reindestillation an die etwa in einer Kolonne, einem Fallfilm oder einem Dünnschichtverdampfer durchgeführt wird. Bei Zusatz einer Säure wird vorzugsweise auf Phosphorsäure zurückgegriffen.

Beispiele

Das erfindungsgemäße Verfahren wird nun anhand der nachfolgenden Beispiele erläutert.

Beispiel 1: TMP-Herstellung

Eine Apparatur bestehend aus zwei beheizbaren, durch Überlaufrohre miteinander verbundenen Rührkesseln mit einem Fassungsvermögen von insgesamt 72 l wurde mit frischer, wäßriger Formaldehydlösung (4300g/l in Form der 40 %igen wäßrigen Lösung und n-Butyraldehyd (1800 g/h) und mit frischem Trimethylamin als Katalysator (130 g/h) in Form der 45 %igen wäßrigen Lösung kontinuierlich beschickt. Der Reaktoren wurden dabei auf 40°C temperiert.

Der Austrag wurde direkt in den oberen Teil eines Fallfilmverdampfers mit aufgesetzter Kolonne (11 bar Heizdampf) geleitet und dort bei normalem Druck destillativ in ein leichtsiedendes Kopfprodukt, im wesentlichen enthaltend n-Butyraldehyd, Ethylacrolein, Formaldehyd, Wasser und Trimethylamin, und ein hochsiedendes Sumpfprodukt aufgetrennt.

Das Kopfprodukt wurde kontinuierlich kondensiert und in die oben beschriebenen Reaktoren zurückgeführt.

Das hochsiedende Sumpfprodukt aus dem Verdampfer (ca. 33,5 kg/h) wurde kontinuierlich mit frischem Trimethylamin-Katalysator (50 g/h, in Form der 45 %igen wäßrigen Lösung) versetzt und in einen beheizbaren, mit Füllkörpern versehenen Rohrreaktor mit einem Leervolumen von 12 l geführt. Der Reaktor war dabei auf 40°C temperiert.

Der Austrag des Nachreaktors wurde kontinuierlich in den oberen Teil einer weiteren Destillationseinrichtung, der Formaldehydabtrennung (11 bar Heizdampf), gegeben und dort destillativ in ein leichtsiedendes Kopfprodukt, im wesentlichen enthaltend Ethylacrolein, Formaldehyd, Wasser und Trimethylamin, und ein hochsiedendes Sumpfprodukt aufgetrennt. Das leichtsiedende Kopfprodukt (27 kg/h) wurde kontinuierlich kondensiert und in den ersten Rührkessel zurückgeleitet, wohingegen das hochsiedende Sumpfprodukt gesammelt wurde.

Das so erhaltene Sumpfprodukt enthielt neben Wasser im wesentlichen Dimethylolbutyraldehyd, Formaldehyd und Spuren Monomethylolbutyraldehyd. Er wurde dann einer kontinuierlichen Hydrierung unterworfen. Dazu wurde die Reaktionslösung bei 90 bar und 115°C in einem Hauptreaktor in Kreislauf/Rieselfahrweise und einem nachgeschalteten Nachreaktor in Kreislauffahrweise hydriert. Der Katalysator wurde analog D der DE 198 09 418 hergestellt. Er enthielt 24 % CuO, 20 % Cu und 46 % $TiO_2$. Die verwendete Apparatur bestand aus einem 10 m langen beheizten Hauptreaktor (Innendurch-messer: 27 mm) und einem 5,3 m langen beheizten Nachreaktor (Innendurchmesser: 25 mm). Der Kreislaufdurchsatz betrug 25 l/h Flüssigkeit, der Reaktorzulauf wurde auf 4 kg/h eingestellt. Dementsprechend wurden 4 kg/h Hydrieraustrag erhalten.

Das nach der Hydrierung erhaltene Gemisch wurde anschließend gemäß der deutschen Anmeldung, mit dem Titel "Verfahren zur Reinigung von durch Hydrierung hergestelltem Trimethylpropan durch kontinuierliche Destillation" (Anmelderin: BASF AG) von Wasser und Trimethylamin befreit. Das Rohgemisch wurde zunächst der Leichtsiederkolonne zugeführt, wobei die Entwässerung bei 400 mbar und 180°C durchgeführt wurde. Der Zulauf erfolgte in der Mitte der Kolonne. Das Rücklaufverhältnis wurde zu 0,3 gewählt. Es wurde ein Sumpfaustrag (1,1 kg/h) erhalten. Der erhaltene Austrag hatte die folgende, in Tabelle 1 ausgegebene, gaschromatographisch ermittelte Zusammensetzung. Für die gaschromatishe Ermittlung der in dieser Anmeldung angegebenen Zusammensetzungen wurde die Säule DB5 der Firma J & W Scientific mit einer Länge von 30 m, einem Durchmesser von 0,32 mm und 1 µm Beschichtung verwendet. Die Detektion erfolgte mit FID.

Vergleichsbeispiel V2

In Beispiel 2 wurde gemäß Beispiel 1 hergestelltes TMP verwendet. Der Sumpfaustrag nach Beispiel 1 wurde durch einen auf Raumtemperatur temperierten 2,6 1-Behälter mit einer Fließgeschwindigkeit von 1,15 l/h - entsprechend einer Verweilzeit von 2,3 h - geleitet und der erhaltene Austrag wurde anschließend wie in DE 199 63 435 beschrieben aufgearbeitet. Dafür wurde zuerst von der Rohlösung mittels einer Hochsiederabtrennung bestehend aus einer Abtriebssäule mit Fallfilmverdampfer bei 20 mbar der Hochsieder-Anteil abgetrennt. Das von Hochsiedern befreite Roh-TMP wurde am Kopf der Kolonne kondensiert. Im Sumpf der Kolonne wurde eine Temperatur von 223°C eingestellt.

Das von Hochsiedern befreite Roh-TMP wurde einer Reindestillation zugeführt, die aus einer mit Abtriebsteil und Verstärkungsteil versehenen Kolonne bestand. Zwischen Wasser und TMP siedende Komponenten wurden über Kopf abgezogen, über Sumpf wurden verbliebene Hochsieder in die Hochsiederabtrennung rückgeführt. Die Reindestillation erfolgte bei einem Druck von 20 mbar und einem Rücklaufverhältnis von 25. Das gereinigte TMP mit der in Tabelle 1 beschriebenen Zusammensetzung wurde am dampfförmigen Seitenabzug direkt oberhalb des Verdampfers gasförmig gewonnen und kondensiert.

Beispiel 3 und 4

Beispiele 3 und 4 werden wie Beispiel 2 durchgeführt, jedoch wurde der 2,8 l-Behälter in Form eines Kolbens oder einer Rohrschlange auf 180°C bzw. 200°C temperiert. Nach der Temperung wurde wie unter Beispiel 2 beschrieben destilliert und die Zusammensetzung gaschromatographisch ermittelt.

Der Gehalt an gebundenem Formaldehyd berechnet sich als Summe des Molgewichtsanteils von Formaldehydäquivalenten an dem jeweiligen Formaldehydacetal, multipliziert mit dessen analytisch gefundenen Anteil am Reaktionsgemisch. Der Formaldehydgehalt, auch FA-Zahl genannt, wurde in Gewichtsprozent anteilsmässig aus den formaldehydhaltigen Acetalen nach der Formel

$$\text{Gehalt (Formaldehyd)} = C_{(\text{TMP-FA-Me})} \cdot \frac{M_{FA}}{M_{\text{TMP-FA-Me}}} + C_{(\text{TMP-diFA-Me})} \cdot \frac{M_{FA}}{M_{\text{TMP-diFA-Me}}} + C_{(\text{cyklTMP-Formal})} \cdot \frac{M_{FA}}{M_{\text{cyklTMP-Formal}}} + C_{(\text{TMP-FA-TMP})} \cdot \frac{M_{FA}}{M_{\text{TMP-FA-TMP}}}$$

wobei

| | |
|---|---|
| TMP-FA-Me | TMP-Formaldehyd-Methanol-Acetal (IIIa) |
| FA | Formaldehyd |
| Me | Methanol |
| TMP-diFA-Me | TMP-DiFormaldehyd-Methanol Acetal (IIIb) |
| cykl. TMP-Formal | cyclisches TMP-Formal (V) |
| C | Konzentration |
| M | Molgewicht |

bedeutet, ermittelt.

Tabelle 1:

| Nr. | | TMP [Gew.-%] | (IIIa) [Gew.-%] | (IIIb) [Gew.-%] | (V) [Gew.-%] | (IV) [Gew.-%] | FA-Zahl [ppm] |
|---|---|---|---|---|---|---|---|
| 1 | Nur Entwässerung | 84,0 | 1,39 | 0,38 | 2,04 | 0,5 | n.b. |
| 2 | Nach Dest. ohne Temperung | 98,9 | 0,1 | 0,31 | 0,05 | 0,1 | 1273 |
| 3 | Temperung 180°C + Destillation | 99,0 | 0,1 | 0,19 | 0,05 | 0,05 | 873 |

(fortgesetzt)

| Nr. | | TMP [Gew.-%] | (IIIa) [Gew.-%] | (IIIb) [Gew.-%] | (V) [Gew.-%] | (IV) [Gew.-%] | FA-Zahl [ppm] |
|---|---|---|---|---|---|---|---|
| 4 | Temperung 200°C + Destillation | 99,2 | 0,02 | 0,09 | 0,02 | 0,05 | 388 |
| IIIa TMP-Di-Formaldehyd-Methanol Acetal<br>IIIb TMP-Formaldehyd-Methanol Acetal<br>IV lineares bis-TMP-Formal<br>V cyclisches TMP-Formal<br>n.b. nicht bestimmt | | | | | | | |

Das in Tabelle 1 zusammengefasste Ergebnis zeigt, dass durch das erfindungsgemäße Verfahren TMP mit Formaldehydgehalten unter 1000 Gew.-ppm, bevorzugt unter 500 Gew.-ppm, zugänglich sind.

## Patentansprüche

1. Verfahren zum Entfernen von formaldehydhaltigen Acetalen aus mehrwertigen Alkoholen, insbesondere Trimethylolpropan, **dadurch gekennzeichnet, dass** der mehrwertige Alkohol nach seiner Herstellung durch Destillation von Leichtsiedern befreit wird, anschließend einer Temperung unterworfen und anschliessend erneut durch Destillation gereinigt wird.

2. Verfahren nach Anspruch 1, **dadurch gekennzeichnet, dass** der in der Temperung eingesetzte mehrwertige Alkohol nach dem Hydrierverfahren hergestellt wurde.

3. Verfahren nach Anspruch 1 oder 2, **dadurch gekennzeichnet, dass** der eingesetzte mehrwertige Alkohol einen Produktgehalt von 60 bis 95 Gew.-% aufweist.

4. Verfahren nach Anspruch 1 oder 2, **dadurch gekennzeichnet, dass** der von Leichtsiedern befreite mehrwertige Alkohol vor der Temperung durch Destillation gereinigt wurde.

5. Verfahren nach Anspruch 4, **dadurch gekennzeichnet, dass** der mehrwertige Alkohol einen Produktgehalt von > 95 Gew.-%, im Fall von Trimethylolpropan von > 98 Gew.-%, aufweist.

6. Verfahren nach einem der Ansprüche 1 bis 5, **dadurch gekennzeichnet, dass** der Temperschritt bei Temperaturen von 100 bis 300°C, Verweilzeiten von 5 min bis 24 h, und Drücken von 100 mbar bis 200 bar durchgeführt wird.

7. Verfahren nach Anspruch 6, **dadurch gekennzeichnet, dass** der mehrwertige Alkohol Trimethylolpropan ist.

8. Verfahren nach einem der Ansprüche 1 bis 7, **dadurch gekennzeichnet, dass** die Temperung diskontinuierlich, vorzugsweise im Rührkessel, oder kontinuierlich, vorzugsweise in Rohrreaktoren in Sumpf- oder Rieselfahrweise, meistbevorzugt in Sumpffahrweise im Rohrreaktor, betrieben wird.

## Claims

1. A process for the removal of formaldehyde-containing acetals from polyhydric alcohols, especially trimethylolpropane, wherein, after it has been prepared, the polyhydric alcohol is separated from low boilers by distillation, then subjected to a tempering and then purified again by distillation.

2. A process according to claim 1 wherein the polyhydric alcohol used in the tempering has been prepared by the hydrogenation process.

3. A process according to claim 1 or 2 wherein the polyhydric alcohol used has a product content of 60 to 95% by weight.

4. A process according to claim 1 or 2 wherein the polyhydric alcohol freed of low boilers has been purified by distillation prior to tempering.

5. A process according to claim 4 wherein the polyhydric alcohol has a product content of >95% by weight or, in the case of trimethylolpropane, of >98% by weight.

6. A process according to one of claims 1 to 5 wherein the tempering step is carried out at temperatures of 100 to 300°C, for residence times of 5 min to 24 h, and at pressures of 100 mbar to 200 bar.

7. A process according to claim 6 wherein the polyhydric alcohol is trimethylolpropane.

8. A process according to one of claims 1 to 7 wherein the tempering is carried out batchwise, preferably in a stirred tank, or continuously, preferably in tube reactors by the sump or trickle procedure and particularly preferably in a tube reactor by the sump procedure.

## Revendications

1. Procédé pour l'élimination d'acétals contenant du formaldéhyde à partir d'alcools polyhydriques, en particulier de triméthylpropane, **caractérisé en ce qu'**après sa production on libère par distillation l'alcool polyhydrique des composants à bas point d'ébullition, puis on le soumet à un traitement thermique et ensuite on le purifie de nouveau par distillation.

2. Procédé selon la revendication 1, **caractérisé en ce que** l'alcool polyhydrique utilisé dans le traitement thermique a été produit selon le procédé d'hydrogénation.

3. Procédé selon la revendication 1 ou 2, **caractérisé en ce que** l'alcool polyhydrique utilisé présente une teneur en produit de 60 à 95 % en poids.

4. Procédé selon la revendication 1 ou 2, **caractérisé en ce qu'**avant le traitement thermique l'alcool polyhydrique libéré des composants à bas point d'ébullition a été purifié par distillation.

5. Procédé selon la revendication 4, **caractérisé en ce que** l'alcool polyhydrique présente une teneur en produit de > 95 % en poids, dans le cas du triméthylpropane de > 98 % en poids.

6. Procédé selon l'une quelconque des revendications 1 à 5, **caractérisé en ce qu'**on effectue l'étape de traitement thermique à des températures de 100 à 300 °C, pendant des temps de séjour de 5 min à 24 h et sous des pressions de 100 mbars à 200 bars.

7. Procédé selon la revendication 6, **caractérisé en ce que** l'alcool polyhydrique est le triméthylol-propane.

8. Procédé selon l'une quelconque des revendications 1 à 7, **caractérisé en ce que** le traitement thermique est conduit en mode discontinu, de préférence en récipient à agitation, ou en continu, de préférence dans des réacteurs tubulaires en mode opératoire à flux de bas en haut ou par ruissellement, de façon tout particulièrement préférée en mode opératoire à flux de bas en haut dans le réacteur tubulaire.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9828253 A
- US 6096905 A
- GB 1290036 A
- DE 19963435
- DE 19809418